(19) **Europäisches Patentamt**

**European Patent Office**

**Office européen des brevets**

(11) **EP 0 491 850 B1**

(12) **EUROPEAN PATENT SPECIFICATION**

(45) Date of publication and mention
of the grant of the patent:
**22.07.1998 Bulletin 1998/30**

(21) Application number: **90914818.1**

(22) Date of filing: **11.09.1990**

(51) Int. Cl.⁶: **B01D 24/02**

(86) International application number:
**PCT/US90/05141**

(87) International publication number:
**WO 91/04088 (04.04.1991 Gazette 1991/08)**

(54) **DEVICE AND METHOD FOR PROCESSING BLOOD FOR HUMAN TRANSFUSION**

VERFAHREN UND VORRICHTUNG ZUR BEHANDLUNG VON BLUT FÜR DIE MENSCHLICHE TRANSFUSION

DISPOSITIF ET PROCEDE DE TRAITEMENT DE SANG POUR UNE TRANSFUSION CHEZ L'HOMME

(84) Designated Contracting States:
**AT BE DE DK FR GB IT NL SE**

(30) Priority: **12.09.1989 US 405977**

(43) Date of publication of application:
**01.07.1992 Bulletin 1992/27**

(60) Divisional application:
**97122051.2**

(73) Proprietor: **PALL CORPORATION**
**Glen Cove, New York 11542 (US)**

(72) Inventors:
• **PALL, David, B.**
**Roslyn Estates, NY 11576 (US)**
• **GSELL, Thomas, C.**
**Glen Cove, NY 11542 (US)**
• **MUELLERS, Brian, T.**
**Rockville Centre, NY 11570 (US)**

(74) Representative:
**Dost, Wolfgang, Dr.rer.nat., Dipl.-Chem. et al**
**Patent- und Rechtsanwälte**
**Bardehle . Pagenberg . Dost . Altenburg .**
**Frohwitter . Geissler & Partner**
**Postfach 86 06 20**
**81633 München (DE)**

(56) References cited:
| | |
|---|---|
| **EP-A- 0 243 744** | **EP-A- 0 329 303** |
| **US-A- 3 513 976** | **US-A- 4 040 959** |
| **US-A- 4 111 199** | **US-A- 4 187 979** |
| **US-A- 4 322 298** | **US-A- 4 330 410** |
| **US-A- 4 416 777** | **US-A- 4 447 220** |
| **US-A- 4 464 167** | **US-A- 4 596 657** |
| **US-A- 4 608 178** | **US-A- 4 657 117** |
| **US-A- 4 663 032** | **US-A- 4 663 058** |
| **US-A- 4 687 580** | **US-A- 4 720 284** |
| **US-A- 4 747 952** | **US-A- 4 767 541** |
| **US-A- 4 776 964** | **US-A- 4 880 548** |
| **US-A- 4 915 848** | |

**Description**

Technical Field:

This invention relates to a method for processing blood donated for the purpose of therapeutic transfusion of blood components and, particularly, to improved means for preparing, from the donated whole blood, leucocyte depleted platelet concentrate (hereinafter PC), as well as a blood component substantially free of red blood cells.

Background Art:

The development of plastic blood collection bags facilitated the separation of donated whole blood into its various components, thereby making platelet concentrates available as a transfusion product. The separation of a single unit of donated whole blood, about 450 milliliter in USA practice, into its components is typically accomplished by use of differential sedimentation.

A typical procedure used in the United States, the citrate-phosphate-dextrose-adenine (CPDA-1) system, utilizes a series of steps to separate donated blood into three components, each component having substantial therapeutic and monetary value. The procedure typically utilizes a blood collection bag to which is integrally attached at least one, and preferably two, satellite bags. Whole blood may be thus collected and processed as follows:

(1) The donated whole blood is collected from the donor's vein directly into the blood collection bag which contains the nutrient and anti-coagulant containing CPDA-1.

(2) The blood collection bag is centrifuged together with its satellite bags, thereby concentrating the red cells as packed red cells (PRC) in the lower portion of the blood collection bag and leaving in the upper portion of the bag a suspension of platelets in clear plasma, which is known as platelet-rich plasma (PRP).

(3) The blood collection bag is transferred, with care not to disturb the interface between the supernatant PRP layer and the sedimented PRC layer, into a device known as a "plasma extractor" which comprises an opaque back plate and a transparent front plate; the two plates are hinged together at their lower ends and spring biased toward each other such that a pressure of about 200 to 300 millimeters of mercury is developed within the bag.

With the blood collection bag positioned between the two plates, a valve is opened allowing the supernatant PRP to flow into a first satellite bag. As the PRP flows out of the blood collection bag, the interface with the PRC rises. The operator closely observes the position of the interface as it rises and clamps off the connecting tube when in his judgment as much PRP has been transferred as is possible, consistent with allowing no red cells to enter the first satellite bag. This is a time consuming operation during which the operator must visually monitor the bag and judiciously and arbitrarily ascertain when to shut-off the connecting tube. The blood collection bag, now containing only PRC, is then detached and stored at 4°C until required for transfusion into a patient.

(4) The PRP-containing satellite bag, together with the second satellite bag, is then removed from the extractor and centrifuged at an elevated G force with the time and speed adjusted so as to concentrate the platelets into the lower portion of the PRP bag. When centrifugation is complete, the PRP bag contains platelet concentrate in its lower portion and clear plasma in its upper portion.

(5) The PRP bag is then placed in the plasma extractor, and the clear plasma is expressed into the second satellite bag, leaving the first bag containing only platelet concentrate (PC). Again, the operator must judiciously and arbitrarily ascertain when to halt plasma flow into the second bag. The PRP bag, now containing PC, is then detached and stored for up to five days at 20°-22°C until needed for a transfusion of platelets. For use with adult patients, the platelets from 6-10 donors are, when required, pooled into a single platelet transfusion.

(6) The plasma in the second satellite bag is usually separated by complex processes into a variety of valuable products.

Commonly used systems, other than CPDA-1, include Adsol and SAG-M. In these systems, nutrient solution is preplaced in the second nutrient bag, and this solution is transferred into the PRC in an additional step after the PRP has been separated from the PRC, thereby achieving a higher yield of plasma and longer storage life for the PRC.

The separation of blood into components has substantial therapeutic and monetary value. This is nowhere more evident than in treating the increased damage to a patient's immune system caused by the higher doses and stronger drugs now used during chemotherapy for cancer patients. These more aggressive chemotherapy protocols are directly implicated in the reduction of the platelet content of the blood to abnormally low levels; associated internal and external bleeding additionally requires more frequent transfusions of PC, and this has caused platelets to be nationally in short supply and has put pressure on the blood banks to increase platelet yield per unit of blood.

Blood bank personnel have responded to this pressure by attempting to increase PC yield in a variety of ways, including attempting to express more PRP prior to stopping flow from the blood collection bag. This has often proved to be counterproductive in that the PRP, and the PC subsequently extracted from it, were not infrequently contaminated by red cells, giving a pink or red color to the normally light yellow PC. The presence of red cells in PC is so highly undesirable that pink or red PC is frequently discarded, or subjected to recentrifugation, both of which increase operating costs.

The devices and methods of this invention alleviate the above-described problems and, in addition, provide a higher yield of superior quality PC.

Leucocyte Depletion of Platelet Suspensions

The transfusion of platelet concentrate which has not been leuco-depleted is not without risk for those patients receiving both acute and chronic transfusion support. Chills, fever, and allergic reactions may occur in patients receiving acute as well as chronic platelet therapy. Repeated platelet transfusions frequently lead to alloimmunization against HLA antigens, as well as platelet specific antigens. This, in turn, decreases responsiveness to platelet transfusion. Leucocytes contaminating platelet concentrates, including granulocytes and lymphocytes, are associated with both febrile reactions and alloimmunization, leading to platelet transfusion refractoriness. Another life-threatening phenomenon affecting heavily immunosuppressed patients is Graft Versus Host Disease. In this clinical syndrome, donor lymphocytes transfused with the platelet preparations can launch an immunological reaction against the transfusion recipient with pathological consequences.

Growing evidence suggests that the use of leucocyte depleted platelet concentrates decreases the incidence of febrile reactions and platelet refractoriness. Leucocyte depleted blood components are also believed to have a role in reducing the potential for Graft Versus Host Disease. Leucocyte depletion of platelet preparations are also believed to diminish, but not to completely prevent, the transmission of leucocyte associated viruses such as HIV-1 and CMV.

Platelet preparations contain varying amounts of leucocytes. Platelet concentrates prepared by the differential centrifugation of blood components will have varying leucocyte contamination related to the time of centrifugation and G force used. While the dose of contaminating leucocytes necessary to cause a febrile reaction or to elicit platelet refractoriness in repeated transfusion remains unknown, several recent studies report a reduction of alloimmunization and platelet refractoriness at levels of leucocyte contamination $< 1 \times 10^7$ per unit. These and other studies suggest that at least a two log (99%) reduction of leucocyte contamination is required. More recent studies suggest that a three log (99.9%) or four log (99.99%) reduction would be significantly more beneficial.

U. S. Patent 4,880,548 (hereinafter '548) provides a convenient and very effective means of leuco-depletion of PC. PC is passed through a fibrous filter which permits recovery of 90% or more of the platelets, which pass through the filter, while retaining within the filter more than 99.9% of the incident leucocytes. This system is currently in widespread use at bedside in hospitals, but, unlike the device of this invention, it is not well suited for use in blood banks during the processing of donated blood. The unsuitability stems primarily from additional storage constraints associated with PC and the methods of administering PC. For example, platelets in PC are typically suspended in a total volume of only about 40 to 60 ml of plasma. Contrasted with this, the platelets which are processed by the devices and methods of this invention are derived from a single unit of whole blood and are suspended as PRP in about 180 to 240 ml of plasma. Further, the platelets in PC have been subjected during centrifugation to severe conditions and there is reason to believe that, as a result of the high forces to which the platelets are subjected as they reach the bottom of the bag during sedimentation, they are not as readily dispersed, i.e., they are more aggregated by particle-to-particle adhesion, when compared with the platelet distribution in PRP.

For these and perhaps other reasons, platelets in PC show a much higher tendency to be retained within the filter during leuco-depletion compared with platelets in PRP. Indeed, one of the advantages of the devices and methods of this invention is that much better recovery is obtained when platelets are leuco-depleted in the form of PRP, compared with PC; for example, while optimal recovery from PC is about 90 to 95%, recovery from PRP can exceed 99%.

EP-A-0 329 303 is related to the separation of leucocytes from platelet concentrate (PC) by passing single, or pooled, units of PC through a leucocyte depletion filter. It does not refer to separating leucocytes from platelet-rich plasma (PRP).

EP-A-0 243 744 is concerned with a filtering system for the removal of white blood cells (leucocytes) from red blood cells. In the method of EP-A-0 243 744 whole blood (contained in blood bag 3) is first centrifuged to separate the blood into denser packed red blood cells and less dense platelet-rich plasma (PRP) which latter is expressed into one of the satellite bags 9, leaving behind in bag 3 the packed red blood cells (RBCs) including both white blood cells (WBCs) and some platelets. The content in bag 3 is then reconstituted, the reconstituted mixture then being passed from bag 3 through filter 15. The result is the removal of leucocytes by filter 15 whereas the red cells pass the filter 15 to be collected in bag 7.

Brief Description of the Drawings:

Figure 1 is a representational view of a typical blood collection set for the collection and processing of blood. The set 10 comprises a collection bag 11 with flexible tubing 12 connected thereto and leading to a first satellite bag 13 (for PRP) and a second satellite bag 15 (for plasma) connected to the first satellite bag 13 via flexible tubing 16.
Figure 2 is identical to Figure 1 except that tubing 12 has been cut and filter 14 inserted.

Disclosure of the Invention:

The invention relates to a device for the processing of blood comprising a first container and at least one second container connected thereto, wherein a filter is interposed between the first container and the second container, the filter permitting platelets to pass therethrough, but blocking flow when red blood cells reach the filter.
In another aspect the invention relates to a device for processing blood comprising a first container and at least one second container connected thereto, wherein a filter is interposed between the first container and the second container, said filter comprising a porous, fibrous medium removing leucocytes and permitting platelet-rich plasma to pass therethrough but blocking flow when red blood cells reach the filter.
In a further aspect the invention relates to a method for producing a blood component substantially free of red blood cells comprising:

centrifuging blood or a blood component in a first container to produce a supernatant layer and a sediment layer, wherein the sediment layer contains red blood cells;

passing the supernatant layer from the first container to a second container through a device including a filter that allows fluid flow therethrough and blocks flow when red blood cells reach the filter, and

collecting the blood component substantially free of red blood cells in the second container.

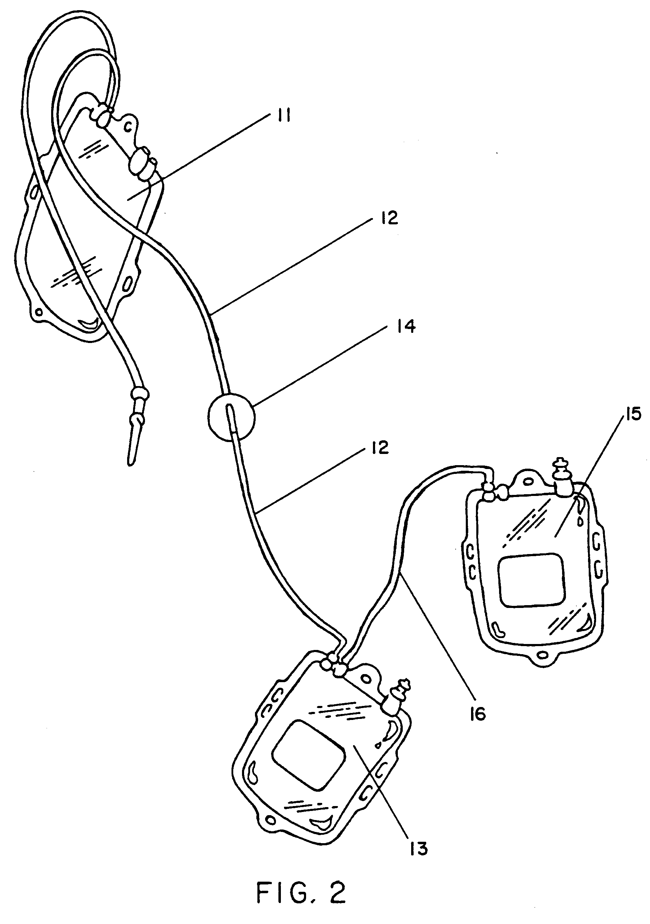

PC is typically prepared from donated blood that has been stored until needed and transfused to the patient at bedside as required. Insofar as leuco-depletion of platelets derived from donated blood has been practiced, it has generally been accomplished just prior to or contemporaneous with the transfusion to the patient.
In the methods of this invention, leucocyte depletion is accomplished at the time the blood is processed, which in USA practice is within 8 hours of the time the blood was drawn. Step number three of the six step process of blood bank practice presented in a preceding section is modified by interposing a leucocyte depletion filter 14 immediately downstream of the blood collection bag 11 (see Figure 2). Thus, as the supernatant PRP is expressed by the plasma extractor, leucocytes are removed by the filter and leucocyte-depleted PRP is collected in the satellite bag and is subsequently centrifuged to obtain leucocyte-depleted PC and plasma.
In a preferred form of the filter of the subject invention, the fibers of which the filter element is composed are modified by grafting thereon a mixture of two monomers, one containing hydroxyl groups and another containing anionic groups, such as carboxyl groups, with the hydroxyl groups present in larger numbers. As described in U. S. Patent 4,880,548, herein incorporated by reference, the filter media of this invention are preferably surface modified using a mixture comprising hydroxyl-terminated and carboxyl-terminated monomers. In a preferred form of this invention, the monomers are respectively hydroxyethyl methacrylate and methacrylic acid, and the monomer ratios are preferably in the range (carboxyl:hydroxyl) of 0.01:1 to 0.5:1, and more preferably in the range of 0.05:1 to 0.35:1. A preferred monomer ratio is one which produces a desired zeta potential at the pH of plasma (7.3) of -3 to -30 millivolts, a more preferred ratio produces a zeta potential of -7 to -20 millivolts, and a still more preferred ratio produces a zeta potential of -10 to -14 millivolts.
The CWST of the filter elements made with the PBT fibers according to this invention have a CWST as formed of about 50 to 54 dynes/cm, and most or all other fibers which may be used have a CWST below 55 dynes/cm. Surface grafting using the monomers noted above causes the CWST of the fibers to increase, the exact value obtained being dependent on the ratio of the two monomers. A preferred range for the CWST of the devices of this invention is about 70 to 115 dynes/cm, a more preferred range is 90 to 100 dynes/cm and a still more preferred range is 93 to 97 dynes/cm, these ranges being obtained by varying the ratio of carboxyl-terminated and hydroxyl-terminated monomers.
Although the fibers of the porous medium may remain untreated, they are preferably treated to make them even more effective. For example, the fibers may be surface modified to increase the critical wetting surface tension (CWST) of the fibers.
As disclosed in U.S. Patent No. 4,880,548, the CWST of a porous medium may be determined by individually applying to its surface a series of liquids with surface tensions varying by 2 to 4 dynes/cm and observing the absorption or non-absorption of each liquid over time. The CWST of a porous medium, in units of dynes/cm, is defined as the mean value of the surface tension of the liquid which is absorbed and that of the liquid of neighboring surface tension which is not absorbed within a predetermined amount of time. The absorbed and non-absorbed values depend principally on the surface characteristics of the material from which the porous medium is made and secondarily on the pore size characteristics of the porous medium.

Liquids with surface tensions lower than the CWST of a porous medium will spontaneously wet the medium on contact and, if the medium has through holes, will flow through it readily. Liquids with surface tensions higher than the CWST of the porous medium may not flow at all at low differential pressures and may do so unevenly at sufficiently high differential pressures to force the liquid through the porous medium. In order to achieve adequate priming of a fibrous medium with a liquid such as blood, the fibrous medium preferably has a CWST in the range of about 53 dynes/cm or higher.

The number of carboxyl groups per unit of surface area appears to have an important effect on the adhesion of platelets to fiber surfaces. This effect is reflected in the proportion of platelets recovered in the filter effluent as a fraction of the number present in the platelets prior to filtration. Platelet recovery peaks at the optimum proportion of methacrylic acid (MAA). The number of carboxyl groups per unit of fiber surface is, over the range of interest of this invention, thought to be close to proportional to the amount of MAA in the monomeric grafting solution.

The porous medium of this invention may be formed, for example, from any synthetic polymer capable of forming fibers and of serving as a substrate for grafting. Preferably, the polymer should be capable of reacting with at least one ethylenically unsaturated monomer under the influence of ionizing radiation without the matrix being adversely affected by the radiation. Suitable polymers for use as the substrate include, but are not limited to, polyolefins, polyesters, polyamides, polysulfones, acrylics, polyacrylonitriles, polyaramides, polyarylene oxides and sulfides, and polymers and copolymers made from halogenated olefins and unsaturated nitriles. Examples include, but are not limited to, polyvinylidene fluoride, polyethylene, polypropylene, cellulose acetate, and Nylon 6 and 66. Preferred polymers are polyolefins, polyesters, and polyamides. The most preferred polymer is polybutylene terephthalate (PBT).

Surface characteristics of a fiber can be modified by a number of methods, for example, by chemical reaction including wet or dry oxidation, by coating the surface by depositing a polymer thereon, and by grafting reactions which are activated by exposure to an energy source such as heat, a Van der Graff generator, ultraviolet light, or to various other forms of radiation. The preferred method is a grafting reaction using gamma-radiation, for example, from a cobalt source.

Radiation grafting, when carried out under appropriate conditions, has the advantage of considerable flexibility in the choice of reactants, surfaces, and in the methods for activating the required reaction. Gamma-radiation grafting is particularly preferable because the products are very stable and have undetectably low aqueous extractable levels. Furthermore, the ability to prepare synthetic organic fibrous media having a CWST within a desired range is more readily accomplished using a gamma radiation grafting technique.

An exemplary radiation grafting technique employs at least one of a variety of monomers each comprising an ethylene or acrylic moiety and a second group, which can be selected from hydrophilic groups (e.g., -COOH, or -OH). Grafting of the fibrous medium may also be accomplished by compounds containing an ethylenically unsaturated group, such as an acrylic moiety, combined with a hydroxyl group, such as, hydroxyethyl methacrylate (HEMA), acrylic acid, or combined with methacrylic acid. Use of HEMA as the monomer contributes to a very high CWST. Analogues with similar functional characteristics may also be used to modify the surface characteristics of fibers.

In a first variation not claimed in the present invention, however described on the next few pages and ending with subparagraphs (a) to (f), the PRP derived from a single unit of about 450 m$\ell$ of human blood is passed, typically during a flow interval of 10 to 40 minutes, through a filter comprising the grafted fibers, the element of the filter preferably comprising fibers with a surface area in the range of 0.15 to 1.0 m$^2$, and more preferably 0.2 to 0.7 m$^2$ with a voids volume in the range of 78% to 89% (i.e., if PBT fiber is used, corresponding to a density of the filter element in the range of 0.15 g/m$\ell$ to 0.30 g/m$\ell$, and more preferably 81% to 85% (for PBT, 0.21 g/m$\ell$ to 0.26 g/m$\ell$. The filter element is preferably of right cylindrical form with the ratio of diameter to thickness preferably in the range of 7:1 to 40:1. The range of fiber diameter is preferred to be from 1.0 to 4$\mu$m and is more preferred to be in the range of 2 to 3$\mu$m. These parameters can be varied; for example, the diameter of the filter element could be reduced and the thickness of the filter element increased while retaining the same total quantity of fiber, or the fibers could be larger in diameter while increasing the total quantity of fiber, or the fibers could be packed as opposed to preformed into a cylindrical disc.

If desired, flow rate of the PRP through the filter can be regulated to obtain a total flow period of 10 to 40 minutes by selecting the appropriate element diameter, element thickness, fiber diameter, and density, and/or by varying the diameter of tube 12 either upstream or downstream of the filter, or both up and downstream. At these flow rates, leucocyte depletion efficiency in excess of about 99.9% may be achieved and even as high as 99.9995%. These levels of efficiency result in a PC product with substantially less than 10$^7$ leucocytes per unit of PC (a unit of PC is the volume of PC obtained from a single unit of donated blood).

The above-described device and its mode of use provide the advantages set forth below, among other advantages.

(a) In the blood bank, the filtration step requires no labor input additional to the current practice, and, in the hospital, the need for bedside filtration is completely eliminated.

(b) The volume of the PRP processed is about five or more times that of the PC which is derived from the PRP. Because the volume processed is larger, loss of PC due to hold up within the filter is only about 1% compared with a loss about five or more times greater when PC is filtered at bedside.

(c) Compared with hospital practice, filtration within the blood bank is generally under better control, as it is performed in relatively larger numbers by personnel trained to the specific task.

(d) It is the belief of some researchers that when PC is stored prior to removal of leucocytes, the platelets are damaged during storage as the leucocytes disintegrate, releasing their components, some of which are highly toxic to human tissues. Removing the leucocytes within a few hours after collection is believed to greatly reduce damage due to this cause.

(e) In the process of tapping the donor's vein, the hypodermic needle cuts a disc of the donor's skin which is transferred into the collected blood. The alcohol swab applied prior to venipuncture is not adequate to assure sterility of this skin disc. Thus, the skin disc may contain one or more varieties of bacteria, the most common being Staphylococcus epidermidis, which has been detected in PC along with other organisms. The presence of the skin disc in PC is a suspect source of bacterial growth during storage, and it is fear of such growth which is the principal impetus for the regulation (in the USA) which limits the storage life of platelets to five days. Removal of the skin disc by filtration at an early stage of processing is, for this reason, an important advantage as it may permit the five day regulation to be relaxed.

(f) Compared with a bedside filtration method of US-A-4 880 548, improved recovery of platelets is obtained, i.e., recovery in excess of 98% to 99% compared with about 90 to 95% typically recovered in bedside filtration.

In a second variation which is in accordance with the present invention, the interposed filter 14 is preferably made with smaller fiber surface area, smaller filter element flow area, higher filter element density, and reduced voids volume in relation to the first variation. The total quantity of fiber used is also reduced such that a preferred range for the fiber surface area of the filter element is 0.04 to 0.3 $m^2$ and a more preferred range is 0.06 to 0.20 $m^2$. A preferred range for the filter element flow area is 3 to 8 $cm^2$, and a more preferred range is 4 to 6 $cm^2$. A preferred range for the relative voids volume is 71% to about 83% (corresponding for PBT fibers to a density of 0.23 to 0.40 g/m$\ell$, and a more preferred range is from 73% to 80% (0.27 to 0.37 g/m$\ell$). A preferred range for the CWST of the fiber is 70 to 115 dynes/cm, a more preferred range is 90 to 100 dynes/cm, and a still more preferred range is 93 to 97 dynes/cm. Because of its very small size, a preferred device in accordance with the second variation of the invention retains internally only 0.5 to 1 m$\ell$ of PRP, representing less than a 0.5% loss of platelets.

Filters made in accordance with this second variation and which are interposed between the blood collection bag and PRP bag will generally remove from 85 to 99% or more of the incident leucocytes. Table II shows that this removal rate is not sufficient to consistently achieve a residual leucocyte count of less than $10^7$ leucocytes per 50 ml of PC. A principal function of this device, however, is to act as an automatic "valve" during the decantation process by instantly stopping the flow of PRP at the moment that red cells contact the filter surface. The mechanism of this valve-like action is not well understood, but it may reflect aggregation of the red cells as they reach the filter surface, forming a barrier which prevents or blocks further flow of PRP through the filter element. Aggregation of red cells on contact with the filter surface appears to be related to the CWST and/or to the surface characteristics of the fibers which are generated by the herein described procedure for modifying the fibers. This theory for the proposed mechanism is supported by the existence of filters capable of highly efficient leucocyte depletion of human red blood cell suspensions and which have pore sizes as small as 0.5μm. In these filters, red cells pass freely and completely through the filters with no clogging, with applied pressure of the same magnitude as that used in the present invention. On the other hand, filters of the present invention, which typically have pore diameters larger than about 0.5μm, abruptly stop the flow of red blood cells when the filter is contacted by the red cells. This suggests that the filter's valve-like action is not related to or caused by pore size or by a filtration mechanism. The mechanism of this valve-like action is not well understood, but it may reflect zeta potential-related aggregation of the red cells as they reach the filter surface, forming a barrier which prevents or blocks further flow of PRP through the filter element.

The advantages to be gained by the use of this device include the following:

(a) The collected PRP, and the PC derived therefrom, are substantially free of red cells.

(b) The operator needs only to start the flow of PRP, which will continue to flow into the first satellite bag until red cells contact the filter surface, at which point flow stops. This eliminates the need for a skilled operator to estimate when to stop flow. The PRP so obtained has the faintly yellow color of normal PRP and, for practical purposes, may be considered to be free of red cells. The PC derived from the PRP has the characteristic light yellow color of PC and, for practical purposes, may be considered to be essentially free of red cells.

(c) The volume of PRP recovered from the blood collection bag during the plasma extraction operation is increased by about 2% to about 3% when compared with very competent manual operation and probably by about 2% to about 5% compared with average blood bank practice.

(d) Labor input is reduced, as monitoring of the interface during decantation is not required.

(e) Freshly donated blood contains platelets varying in age from newly formed to nine days or more (platelet half-life in vivo is about nine days). Newly formed platelets are larger and are generally believed to be more active. Because the younger platelets are larger, they tend to sediment faster during centrifugation and, consequently, are present in larger numbers in the PRP nearest to the red cell interface. Measurements have shown that the concentration of platelets in the 10% of the PRP volume nearest the interface is about twice that in the uppermost 10% of PRP. Taking this into account, the total number of platelets recovered is increased by about 4 to 10%.

|  | Incremental number of platelets, % |
|---|---|
| Due to increased volume of PRP | 2 to 5 |
| Due to the higher concentration of platelets in the incremental volume of PRP | 2 to 5 |
| Total | 4 to 10% |

(f) The larger proportion of younger platelets in the PC administered to the patient means that their life within the patient after administration will be longer and that the platelets will be more active, compared with current blood bank practice.

(g) The yield of plasma, a component of value comparable with that of PRC and PC, is also increased by about 2 to about 5%.

(h) Insofar as the plasma yield is increased, the plasma content of the PRC is decreased. This is advantageous because the MHC (major histocompatibility complex) contained in the plasma is responsible for the occurrence of Urticaria (hives) in a proportion of transfusion recipients transfused with PRC.

In a third variation, also in accordance with this invention, the fiber is surface modified as for the preceding versions, but the fiber surface area of the element is increased while, at the same time, the density of the filter element is somewhat reduced. In this way, all of the advantages of the preceding variations are provided together with higher efficiency of leucocyte depletion.

A preferred range of fiber surface area for the third variation of the invention is from 0.3 to 2.0 $m^2$, and a more preferred range is from 0.35 to 0.6 $m^2$. The upper limits of fiber surface area reflect the desire to accomplish the filtration in a relatively short time period, and may be increased if longer filtration times are acceptable. A preferred voids volume of a filter for a filter element is in the range of 71% to 83% (i.e., if PBT fiber is used, corresponding to a density of the filter element in the range of 0.24 g/m$\ell$ to 0.40 g/m$\ell$), and more preferably 75% to 80% (for PBT, 0.28 g/m$\ell$ to 0.35 g/m$\ell$. A preferred filter element flow area is from 2.5 to 10 $cm^2$, and a more preferred area is from 3 to 6 $cm^2$. Leucocyte depletion efficiencies in excess of about 99.9 to 99.99%, which corresponds to an average residual leucocyte content per unit of less than about .005 x $10^7$, can be obtained.

Conversion of density when using fibers other than PBT

In the preceding exposition the term density has been used, and the density values quoted for the filter element have been based on the use of PBT fibers. Other fibers which differ in density from the PBT may be used, as noted above, providing that their surfaces have, or have been modified to have, the characteristics noted above, e.g., a CWST of greater than 70 dynes/cm. In accordance with the invention, to use an alternate fiber of different density, the density of an element made using an alternate fiber may be calculated as follows:

Denoting V as a percentage of the voids volume relative to the apparent volume of the PBT element [i.e., V = (volume of voids/volume of element) x 100], the objective is to calculate the element density of an alternate fiber element which will have a relative voids volume percentage equal to V.

If F is the density of the alternate fiber and 1.38 g/cc is taken as the density of PBT fiber, and $M_1$ is the element density of the PBT element and $M_2$ is the density required for an element with equivalent performance, then voids volume V of the PBT fiber element is

$$V = (1 - M_1/1.38) \times 100$$

and the density required for the element made using the alternate fiber is

$$M_2 = F (1 - V/100).$$

The more preferred fiber diameter range for the practice of this invention is about 2 to 3 $\mu$m, the diameter being defined in terms of surface area, as described in U. S. Patent 4,880,548. This range is preferred because much above this range, the dimensions of the elements and consequently the liquid hold-up volumes of the filters become significantly larger; below this range, the filter elements become relatively less coherent and are more easily compressed. For example, an element made using less than 2 $\mu$m polypropylene fibers would be compressed by the pressure developed by the plasma extractor, which can be as high as 300 mm of Hg.

Pore diameters of filter elements in accordance with the invention can be determined using the modified OSU F2 method as described in U. S. Patent 4,925,572.

In accordance with the invention, a useful technique for the measurement of fiber surface area, for example by nitrogen gas adsorption, is that developed by Brunauer, Emmet, and Teller in the 1930's, often referred to as the "BET" measurement. Using PBT as an example, the surface area of melt blown webs can be used to calculate average fiber diameter:

$$\text{Total volume of fiber in 1 gram} = \frac{1}{1.38} \, m\ell$$

(where 1.38 = fiber density of PBT, g/m$\ell$)
hence

$$\frac{\pi d^2 L}{4} = \frac{1}{1.38} \qquad (1)$$

$$\text{Area of the fiber is } \pi dL = A_f \qquad (2)$$

Dividing (1) by (2),

$$\frac{d}{4} = \frac{1}{1.38 A_f} \text{ and } d = \frac{4}{1.38 A_f} = \frac{2.9}{A_f}, \text{ or } (0.345 A_f)^{-1}$$

where L = total length of fiber per gram, d = average fiber diameter in centimeters, and $A_f$ = fiber surface area in cm$^2$/g. If the units of d are micrometers, the units of $A_f$ become m$^2$/g (square meters/gram), which will be used hereinafter. For fibers other than PBT, substitute the density for 1.38.

Examples

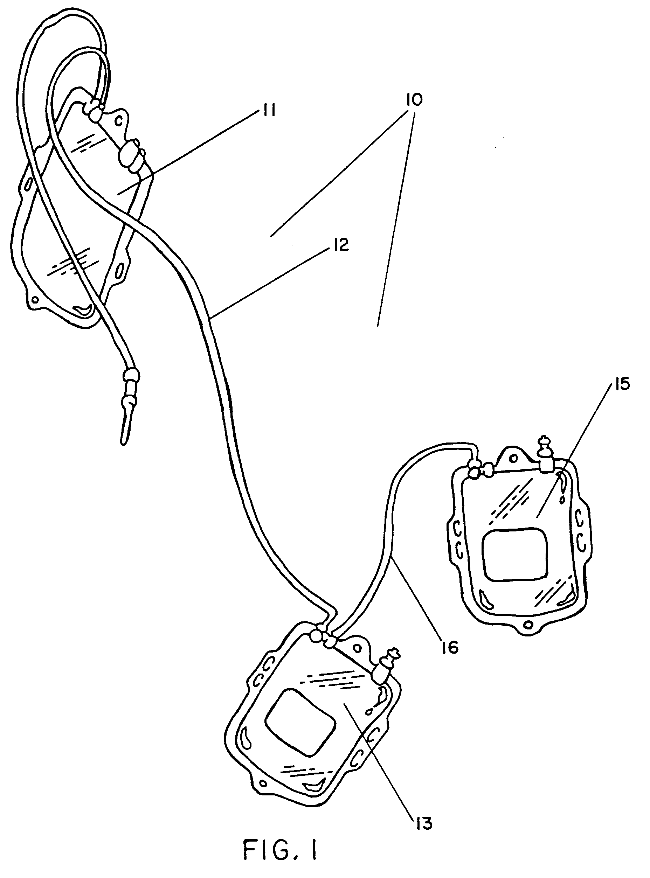

Examples 1 and 2 are in accordance with the first variation not claimed in the present invention, however believed to be necessary for understanding the present invention. Each of the examples was run using the following basic procedure to process and test a bag of donated blood. The blood collection set was constituted as shown in Figure 1. Bag 11, into which anticoagulant had been placed, was used to collect one unit of about 450 m$\ell$ of blood from a human volunteer. Bag 11 along with its two satellite bags was then centrifuged for 5 minutes at 2280 X gravity, causing the red cells to sediment into the lower parts of the bag and leave a transparent, yellowish layer of red cell-free plasma in the upper part of the bag. This bag was then transferred, with care not to disturb its contents, to a plasma extractor. With tube 12 clamped adjacent to bag 11 to prevent flow, tube 12 was cut and the test filter was inserted at position 14 in Figure 2. With the plasma extractor applying sufficient force to the bag to generate a pressure of about 200 to 300 millimeters of mercury within the bag, the clamp on tube 12 was removed, allowing the supernatant liquid to flow through the filter into bag 13 which had been placed on a weight scale. One of several skilled operators was instructed to signal when, in normal blood bank practice, flow would have been manually shut off. For examples 1 and 2, which were in accordance with the first variation, tube 12 was at the signal promptly shut-off, the weight of PRP collected was recorded, and the contents of the bag analyzed, with results recorded in Table I.

For examples 3-8 and 9-10, the weight of the PRP bag 13 was recorded at the signal, i.e., the precise moment when flow would in normal blood bank practice have been shut off, while flow was allowed to continue until the red cell layer reached filter 14, at which time flow spontaneously and abruptly stopped, and the weight of PRP collected was recorded. The results for examples 3-8 are shown in Table II, and for examples 9 and 10 in Table III.

In each of the ten examples, the resulting PRP was visually free of red cells, and weights of the PRP were converted to volume by dividing by the density of plasma (1.04 g/m$\ell$). The data on residual leucocyte content of the PC derived from the filtered PRP are reported in Tables II and III as multiples of $10^7$ (i.e., X $10^7$), which can be conveniently compared with a target criterion of fewer than about 1 X $10^7$ leucocytes per unit, which is a level believed adequate to significantly reduce alloimmunization in patients receiving platelet transfusions.

The widely used melt blowing process for making fibrous plastic webs is a convenient, economical, and effective means for manufacturing fibrous webs with fiber diameter in the 1 - 4μm range. It is characteristic of this process that the quality of melt blown webs is optimal when the web weight is maintained in a preferred range of 0.0005 to 0.01 g/cm$^2$, and more preferably between 0.0005 and 0.007 g/cm$^2$. For this reason, the webs used to form the examples of this invention were, wherever necessary, formed by laying up two or more layers of web of weight about 0.006 g/cm$^2$, and then hot compressing these to form an integral filter element.

Examples 1-2

Devices were prepared in the manner of the first variation. The filter elements of these devices were preformed from 2.6μm average diameter PBT fibers, which had been surface modified in the manner as described above and as taught in U.S. Patent 4,880,548 using a mixture of hydroxyethyl methacrylate and methacrylic acid in a monomer ratio of 0.35:1 to obtain a CWST of 95 dynes/cm and a zeta potential of -11.4 millivolts. Filter element effective diameter was 4.74 cm, presenting a filter area of 17.6 cm$^2$, thickness was 0.15 cm, voids volume was 83% (density = 0.23 g/m$\ell$, and fiber surface area was 0.69 m$^2$. The volume of PRP held up within the filter housing was 2.5 m$\ell$, representing a loss of PRP due to hold-up of about 1%. The results, obtained using the operating procedure described earlier in this section for the first variation, are shown in Table I.

TABLE I

| Leucocyte Depletion Efficiency of the First Variation | | | |
|---|---|---|---|
| Example Number | Volume of PRP passed, m$\ell$ | Leucocyte content of PC after filtration (per unit)* | Leucocyte removal efficiency,** % |
| 1 | 237 | <0.006 x $10^7$ | >99.9% |
| 2 | 206 | <0.006 x $10^7$ | >99.9% |

*Total leucocyte count in the PC after centrifuging the filtered PRP to obtain the PC.
** Assumes that the leucocyte content of the PRP prior to filtration conformed to an average value of 5 x $10^7$ per unit.

Examples 3-8

Devices were prepared in the manner of the second ("automatic valve") variation of this invention. The filter elements of these devices were preformed from 2.6μm average diameter PBT fibers, which had been surface modified in the manner as described above and as taught in U. S. Patent 4,880,548 using hydroxyethyl methacrylate and methacrylic acid in a monomer ratio of 0.35:1 to obtain a CWST of 95 dynes/cm and a zeta potential of -11.4 millivolts. The filter element's effective diameter was 2.31 cm, presenting a filter area of 4.2 cm$^2$, thickness was 0.051 cm, voids volume was 75% (density, 0.34 g/m$\ell$), and fiber surface area was 0.08m$^2$.

The volume of PRP held up within the filter housing was <0.4 m$\ell$, representing a loss of PRP due to hold-up of less than 0.2%. In each test, flow stopped abruptly as red cells reached the upstream surface of the filter element, and there was no visible evidence of red cells or hemoglobin downstream. The results obtained, using the operating procedure described earlier in this section for the second variation, are shown in Table II.

TABLE II

| 1 | 2 | 3 | 4 | 5 |
|---|---|---|---|---|
| Example Number | Estimated volume/PRP using normal blood bank practice, ml | Volume of PRP obtained using the procedure of invention, ml | Incremental volume, percent | Leucocyte content after filtration (per unit) of PC* x $10^7$ |
| 3 | 175.2 | 178.8 | 2.0 | 1.0 |
| 4 | 212.9 | 218.8 | 2.7 | 1.7 |
| 5 | 221.1 | 225.7 | 2.0 | 0.5 |
| 6 | 185.9 | 191.4 | 2.9 | 0.2 |
| 7 | 257.2 | 263.2 | 2.3 | <0.1 |
| 8 | 196.6 | 200.7 | 2.1 | 0.1 |

\* Total leucocyte count in the PC after centrifuging the filtered PRP to obtain PC.

### Examples 9-10

Devices were prepared in the manner of the third variation of this invention, i.e., the combination of an automatic shut-off valve and a high efficiency filter, both comprised in a single filter. The filter elements of these devices were preformed from 2.6μm average diameter PBT fibers, which had been surface modified in the manner as described above and as taught in U.S. Patent 4,880,548 using a mixture of hydroxyethyl methacrylate and methacrylic acid in a monomer ratio of 0.35:1 to obtain a CWST of 95 dynes/cm and a zeta potential of -11.4 millivolts at the pH of plasma (7.3). The filter element effective diameter was 2.31 cm presenting a filter area of 4.2 cm² thickness was 0.305 cm, density was 0.31 g/mℓ (voids volume = 77.5%), and fiber surface area was 0.46 m². The volume of PRP held up within the filter housing was 1.3 mℓ, representing a loss of PRP due to hold up within the filter of about 0.5%. In each case, flow stopped abruptly as red cells reached the upstream surface of the filter element, and there was no visible evidence of red cells or hemoglobin downstream. The results obtained, using the operating procedure described earlier in this section for the third variation, are shown in Table III.

TABLE III

| Incremental Volume and Leucocyte Depletion Efficiency of the Third Variation | | | | | |
|---|---|---|---|---|---|
| Example Number | Estimated volume/PRP using normal blood bank practice, ml | Volume of PRP obtained using the procedure of invention, ml | Incremental volume, % | Leucocyte content after filtration (per unit) of PC* x $10^7$ | Leucocyte removal efficiency** |
| 9 | 251 | 256 | 2 | <0.004 | >99.9% |
| 10 | 212 | 216 | 1.9 | 0.005 | >99.9% |

\* Total leucocyte count in the PC after centrifuging the filtered PRP to obtain PC.
\*\* Assumes that the leucocyte content of the PRP prior to filtration conformed to an average value of 5 x $10^7$ per unit.

## Claims

1. A device for the processing of blood comprising a first container (11) and at least one second container (13) connected thereto, wherein a filter (14) is interposed between the first container (11) and the second container (13), the filter (14) permitting platelets to pass therethrough, but blocking flow when red blood cells reach the filter (14).

2. The device of claim 1 wherein fibers of the filter (14) have been modified to present hydroxyl groups and carboxyl groups.

3. The device of claim 2 wherein the fibers of the filter (14) have been modified with a mixture of monomers comprising hydroxyethyl methacrylate and methacrylic acid.

4. The device of claim 3 wherein the acid/acrylate monomer weight ratio in the modifying mixture is between 0.01:1 to 0.5:1.

5. The device of claim 1 wherein the fibers of the filter medium have a critical wetting surface tension (CWST) from 93 to 97 dynes/cm.

6. The device of claim 1 in which the zeta potential of the fibers of the filter medium is -3 to -30 millivolts at pH of 7.3.

7. The device of claim 5 wherein the fibers comprise polybutylene terephthalate.

8. The device of claim 1 wherein the hold-up volume is less than 1 ml.

9. A device for processing blood comprising a first container (11) and at least one second container (13) connected thereto, wherein a filter (14) is interposed between the first container (11) and the second container (13), said filter (14) comprising a porous, fibrous medium removing leucocytes and permitting platelet-rich plasma to pass therethrough, but blocking flow when red blood cells reach the filter (14).

10. The device of claim 9 wherein said fibrous medium has a critical wetting surface tension (CWST) of 70-115 dynes/cm.

11. The device of claim 9 wherein the zeta potential of the filter medium is -7 to -20 millivolts at pH of 7.3.

12. The device of claim 9 wherein the surface area of the filter medium is 0.3 to 2.0 $m^2$.

13. The device of claim 12 wherein the voids volume of the filter (14) is 71 to 83%.

14. The device of claim 9 wherein the flow area of the filter (14) is 2.5-10 $cm^2$.

15. The device of claim 9 wherein the fibrous medium comprises polybutylene therephthalate fibers.

16. The device of any of the preceding claims, wherein the first container (11) is a blood collection bag and the second container (13) is a satellite bag.

17. The device of any of the preceding claims further comprising a third container (15), wherein the first, second and third containers (11, 13, 15), and the filter (14) are integrally attached.

18. The device of any of claims 2 - 5, 7 and 9 - 15, wherein the fibers have an average fiber diameter in the range of from 2 to 3 $\mu$m.

19. A method for producing a blood component substantially free of red blood cells comprising:

   centrifuging blood or a blood component in a first container (11) to produce a supernatant layer and a sediment layer, wherein the sediment layer contains red blood cells;

   passing the supernatant layer from the first container (11) to a second container (13) through a device including a filter (14) that allows fluid flow therethrough and blocks flow when red blood cells reach the filter, and

   collecting the blood component substantially free of red blood cells in the second container (13).

20. The method of claim 19 wherein the sediment layer comprises packed red blood cells.

21. The method of claim 19 wherein the supernatant layer comprises platelet-rich plasma.

22. The method of any of claims 19 - 21 including centrifuging whole blood in the first container (11) to produce a supernatant layer containing platelets and a sediment layer containing red blood cells.

23. The method of any of claims 19 - 22 wherein the filter depletes leucocytes from the fluid passing through the filter (14).

24. The method of any of claims 19 - 23, including obtaining blood in the first container (11), wherein the method is carried out within 8 hours of obtaining the blood in the first container (11).

25. The method of any of claims 19 - 24, further comprising centrifuging the blood component substantially free of red blood cells in the second container (13) to produce a supernatant layer including plasma, and a sediment layer including concentrated platelets.

26. The method of claim 25 further comprising passing the supernatant layer into a third container (15).

27. The method of claim 26 including centrifuging the blood in the first container (11), producing the blood component substantially free of red blood cells in the second container (13), and passing the supernatant layer into the third container (15), wherein the containers are integrally attached.

**Patentansprüche**

1. Vorrichtung zur Verarbeitung von Blut, umfassend einen ersten Behälter (11) und mindestens einen damit verbundenen zweiten Behälter (13), wobei ein Filter (14) zwischen dem ersten Behälter (11) und dem zweiten Behälter (13) angeordnet ist, wobei der Filter (14) den Durchgang von Blutplättchen ermöglicht, aber den Durchstrom blockiert, wenn rote Blutkörperchen den Filter (14) erreichen.

2. Vorrichtung nach Anspruch 1, wobei die Fasern des Filters (14) so modifiziert sind, daß sie Hydroxylgruppen und Carboxylgruppen aufweisen.

3. Vorrichtung nach Anspruch 2, wobei die Fasern des Filters (14) mit einem Gemisch aus Monomeren mit einem Gehalt an Hydroxyethylmethacrylat und Methacrylsäure modifiziert sind.

4. Vorrichtung nach Anspruch 3, wobei das Gewichtsverhältnis von Säure/Acrylat-Monomeren im modifizierenden Gemisch 0,01:1 bis 0,5:1 beträgt.

5. Vorrichtung nach Anspruch 1, wobei die Fasern des Fasermediums eine kritische Benetzungsoberflächenspannung von 93 bis 97 dyn/cm aufweisen.

6. Vorrichtung nach Anspruch 1, wobei die Fasern des Filtermediums ein zeta-Potential von -3 bis -30 mV beim pH-Wert 7,3 aufweisen.

7. Vorrichtung nach Anspruch 5, wobei die Fasern Polybutylenterephthalat umfassen.

8. Vorrichtung nach Anspruch 1, wobei das Rückhaltevolumen weniger als 1 ml beträgt.

9. Vorrichtung zur Verarbeitung von Blut, umfassend einen ersten Behälter (11) und mindestens einen damit verbundenen zweiten Behälter (13), wobei ein Filter (14) zwischen dem ersten Behälter (11) und dem zweiten Behälter (13) angeordnet ist, wobei der Filter (14) ein poröses, faseriges Medium zur Entfernung von Leukozyten umfaßt und den Durchgang eines an Blutplättchen reichen Plasmas ermöglicht, jedoch den Strom blockiert, wenn rote Blutkörperchen den Filter (14) erreichen.

10. Vorrichtung nach Anspruch 9, wobei das faserige Medium eine kritische Benetzungsoberflächenspannung (CWST) von 70-115 dyn/cm aufweist.

11. Vorrichtung nach Anspruch 9, wobei das zeta-Potential des Filtermediums -7 bis -20 mV beim pH-Wert 7,3 beträgt.

12. Vorrichtung nach Anspruch 9, wobei die Oberfläche des Filtermediums 0,3 bis 2,0 $m^2$ beträgt.

13. Vorrichtung nach Anspruch 12, wobei das Hohlraumvolumen des Filters (14) 71 bis 83% beträgt.

14. Vorrichtung nach Anspruch 9, wobei die Strömungsfläche des Filters (14) 2,5-10 $cm^2$ beträgt.

15. Vorrichtung nach Anspruch 9, wobei das faserige Medium Polybutylenterephthalatfasern umfaßt.

16. Vorrichtung nach einem der vorstehenden Ansprüche, wobei es sich beim ersten Behälter (11) um einen Blutsammelbeutel und beim zweiten Behälter (13) um einen Satellitenbeutel handelt.

17. Vorrichtung nach einem der vorstehenden Ansprüche, ferner umfassend einen dritten Behälter (15), wobei der erste, zweite und dritte Behälter (11, 13, 15) und der Filter (14) in integrierter Bauweise angebracht sind.

18. Vorrichtung nach einem der Ansprüche 2-5, 7 und 9-15, wobei die Fasern einen durchschnittlichen Faserdurchmesser im Bereich von 2 bis 3 μm aufweisen.

19. Verfahren zur Herstellung einer Blutkomponente, die im wesentlichen frei von roten Blutkörperchen ist, umfassend:

das Zentrifugieren von Blut oder einer Blutkomponente in einem ersten Behälter (11) unter Bildung einer überstehenden Schicht und einer Sedimentschicht, wobei die Sedimentschicht rote Blutkörperchen enthält;
das Durchleiten der überstehenden Schicht aus dem ersten Behälter (11) in einen zweiten Behälter (13) unter Durchlaufen einer Vorrichtung, die einen Filter (14) umfaßt, der einen Durchgang eines Flüssigkeitsstroms ermöglicht und den Strom blockiert, wenn rote Blutkörperchen den Filter erreichen; und
das Sammeln der Blutkomponente, die im wesentlichen frei von roten Blutkörperchen ist, im zweiten Behälter (13).

20. Verfahren nach Anspruch 19, wobei die Sedimentschicht gepackte rote Blutkörperchen umfaßt.

21. Verfahren nach Anspruch 19, wobei der Überstand ein an Blutplättchen reiches Plasma umfaßt.

22. Verfahren nach einem der Ansprüche 19-21, umfassend das Zentrifugieren von Vollblut im ersten Behälter (11) unter Bildung einer überstehenden Schicht mit einem Gehalt an Blutplättchen und einer Sedimentschicht mit einem Gehalt an roten Blutkörperchen.

23. Verfahren nach einem der Ansprüche 19-22, wobei der Filter eine Verarmung an Leukozyten aus der den Filter (14) durchlaufenden Flüssigkeit bewirkt.

24. Verfahren nach einem der Ansprüche 19-23, umfassend das Erhalten von Blut im ersten Behälter (11), wobei das Verfahren innerhalb von 8 Stunden nach Erhalten des Bluts im ersten Behälter (11) durchgeführt wird.

25. Verfahren nach einem der Ansprüche 19-24, ferner umfassend das Zentrifugieren der Blutkomponente, die im wesentlichen frei von roten Blutkörperchen ist, im zweiten Behälter (13) unter Bildung einer überstehenden Schicht mit einem Gehalt an Plasma und einer Sedimentschicht mit einem Gehalt an konzentrierten Blutplättchen.

26. Verfahren nach Anspruch 25, ferner umfassend das Durchleiten der überstehenden Schicht in einen dritten Behälter (15).

27. Verfahren nach Anspruch 26, umfassend das Zentrifugieren des Blutes im ersten Behälter (12), das Bilden der Blutkomponente, die im wesentlichen frei von roten Blutkörperchen ist, im zweiten Behälter (13) und das Durchleiten der überstehenden Schicht in den dritten Behälter (15), wobei die Behälter in integrierter Bauweise angebracht sind.

**Revendications**

1. Dispositif pour traiter du sang comportant un premier conteneur (11) et au moins un deuxième conteneur (13) relié à celui-ci, un filtre (14) étant interposé entre le premier conteneur (11) et le deuxième conteneur (13), le filtre (14) permettant à des thrombocytes de passer à travers celui-ci, mais bloquant l'écoulement lorsque des globules rouges atteignent le filtre (14).

2. Dispositif selon la revendication 1, dans lequel des fibres du filtre (14) ont été modifiées pour présenter des groupes hydroxyle et des groupes carboxyle.

3. Dispositif selon la revendication 2, dans lequel les fibres du filtre (14) ont été modifiées à l'aide d'un mélange de monomères constitué de méthacrylate d'hydroxyéthyle et d'acide méthacrylique.

4. Dispositif selon la revendication 3, dans lequel le rapport en poids des monomères acide/acrylate dans le mélange modifiant est situé entre 0,01:1 et 0,5:1.

5. Dispositif selon la revendication 1, dans lequel les fibres du support filtrant ont une tension superficielle de mouillage critique (CWST) allant de 93 à 97 dynes/cm.

6. Dispositif selon la revendication 1, dans lequel le potentiel zêta des fibres du support filtrant est situé entre - 3 et - 30 millivolts à un pH de 7,3.

7. Dispositif selon la revendication 5, dans lequel les fibres sont constituées de polybutylène téréphtalate.

8. Dispositif selon la revendication 1, dans lequel le volume mort est inférieur à 1 ml.

9. Dispositif pour traiter du sang comportant un premier conteneur (11) et au moins un deuxième conteneur (13) relié à celui-ci, un filtre (14) étant interposé entre le premier conteneur (11) et le deuxième conteneur (13), ledit filtre (14) comportant un support fibreux et poreux éliminant les leucocytes et permettant à un plasma riche en thrombocytes de passer à travers celui-ci, mais bloquant l'écoulement lorsque des globules rouges atteignent le filtre (14).

10. Dispositif selon la revendication 9, dans lequel ledit support fibreux a une tension superficielle de mouillage critique (CWST) située entre 70 et 115 dynes/cm.

11. Dispositif selon la revendication 9, dans lequel le potentiel zêta du support filtrant est situé entre - 7 et - 20 millivolts à un pH de 7,3.

12. Dispositif selon la revendication 9, dans lequel la surface superficielle du support filtrant est située entre 0,3 et 2,0,m$^2$.

13. Dispositif selon la revendication 12, dans lequel le volume des vides du filtre (14) est situé entre 71 et 83 %.

14. Dispositif selon la revendication 9, dans lequel la surface d'écoulement du filtre (14) est située entre 2,5 et 10 cm$^2$.

15. Dispositif selon la revendication 9, dans lequel le support fibreux est constitué de fibres de polybutylène téréphtalate.

16. Dispositif selon l'une quelconque des revendications précédentes, dans lequel le premier conteneur (11) est un sac de prélèvement de sang et le deuxième conteneur (13) est un sac satellite.

17. Dispositif selon l'une quelconque des revendications précédentes, comportant de plus un troisième conteneur (15), les premier, deuxième et troisième conteneurs (11, 13, 15) et le filtre (14) étant reliés en un seul bloc.

18. Dispositif selon l'une quelconque des revendications 2 à 5, 7 et 9 à 15, dans lequel les fibres ont un diamètre moyen de fibre situé dans la plage allant de 2 à 3 µm.

19. Procédé de production d'un composant sanguin pratiquement exempt de globules rouges comportant les étapes consistant à :

centrifuger du sang ou un composant sanguin dans un premier conteneur (11) pour produire une couche de surnageant et une couche de sédiment, la couche de sédiment contenant des globules rouges,
faire passer la couche de surnageant du premier conteneur (11) au deuxième conteneur (13) à travers un dispositif comportant un filtre (14) qui permet un écoulement de fluide à travers celui-ci et bloque l'écoulement lorsque des globules rouges atteignent le filtre, et
recueillir le composant sanguin pratiquement exempt de globules rouges dans le deuxième conteneur (13).

20. Procédé selon la revendication 19, dans lequel la couche de sédiment comporte des globules rouges tassés.

21. Procédé selon la revendication 19, dans lequel la couche de surnageant comporte du plasma riche en thrombocytes.

22. Procédé selon l'une quelconque des revendications 19 à 21, consistant à centrifuger tout le sang dans le premier conteneur (11) pour produire une couche de surnageant contenant des thrombocytes et une couche de sédiment contenant des globules rouges.

23. Procédé selon l'une quelconque des revendications 19 à 22, dans lequel le filtre élimine des leucocytes du fluide passant à travers le filtre (14).

24. Procédé selon l'une quelconque des revendications 19 à 23, consistant à obtenir du sang dans le premier conteneur (11), le procédé étant effectué en 8 heures pour obtenir le sang dans le premier conteneur (11).

25. Procédé selon l'une quelconque des revendications 19 à 24, consistant de plus à centrifuger le composant sanguin pratiquement exempt de globules rouges dans le deuxième conteneur (13) afin de produire une couche de surnageant comportant du plasma, et une couche de sédiment comportant des thrombocytes concentrés.

26. Procédé selon la revendication 25, consistant de plus à faire passer la couche de surnageant dans un troisième conteneur (15).

27. Procédé selon la revendication 26, consistant à centrifuger le sang dans le premier conteneur (11), produire le composant sanguin pratiquement exempt de globules rouges dans le deuxième conteneur (13), et faire passer la couche de surnageant dans le troisième conteneur (15), les conteneurs étant reliés en un seul bloc.

FIG. 1

FIG. 2